(19) Europäisches Patentamt / European Patent Office / Office européen des brevets

(11) **EP 4 576 115 A1**

(12) **EUROPEAN PATENT APPLICATION**

(43) Date of publication:
**25.06.2025 Bulletin 2025/26**

(21) Application number: **23218952.2**

(22) Date of filing: **21.12.2023**

(51) International Patent Classification (IPC):
**G16H 40/60** $^{(2018.01)}$    **A61B 5/00** $^{(2006.01)}$
**H04W 56/00** $^{(2009.01)}$

(52) Cooperative Patent Classification (CPC):
**G16H 40/60; A61B 5/0006; A61B 5/0024;
A61B 5/0205; A61B 5/0816; H04W 56/0015;**
G16H 40/63; G16H 40/67

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Novosense AB
223 63 Lund (SE)**

(72) Inventors:
• **TILLY, Jonas
234 40 Lomma (SE)**
• **SEBELIUS, Fredrik
247 34 Södra Sandby (SE)**

(74) Representative: **AWA Sweden AB
Box 5117
200 71 Malmö (SE)**

(54) **WIRELESS SYNCHRONIZATION**

(57) Method of performing a wireless synchronization between a master node and at least one group of a vital sign sensors in a sampling measurement arrangement, each group of vital sign sensors comprising at least one sensor unit, the method comprising sending out, by said master node, a beacon message; for each sensor: receiving, by a receiver, said beacon message, controlling, based on said beacon message, by a control unit, a sampling event in said sensor.

Fig.3

**Description**

Field of the invention

The application relates to the field of wireless vital sign measurement arrangements, and specifically to a system and a method for wireless synchronization of a vital sign measurement arrangement.

Background art

Vital sign sensors are used to collect measurable biological characteristics from a human being. The type of sensor used depends on which vital sign is to be measured. Vital sign sensors are devices that can transduce vital signs into measurable electric signals. The measurement from vital sign sensors can be used within medical diagnosis, treatment and monitoring.

Because vital sign sensors are used within healthcare, it is important that the sensors can provide accurate continuous measurements. To make observations regarding the patient the vital sign sensors samples the electric signal from the transduced electrical signal.

The time when the sample is taken is essential especially for vital signs that are rapidly changing e.g. ECG or when multiple sensors are used.

Synchronization of the sensors is important in order to obtain accurate measurements in order to perform accurate analyses of the measurements.

A conversion of an electrical signal is done by using an analog-to-digital converter ADC. The ADC needs a clock signal for timing the conversion and also a sampling triggering event, to control the sampling frequency. The timing required is accomplished by using an oscillator, one or several counters which will trigger an event when reaching a set value called catch and compare. The oscillator provided the main clock signal, which could be divided down by using counters that increment the counter value for each incoming period. Counters can be connected in series, and you can relay output from specific registers to relay the divided clock. The counter could be converted to a timer by adding a register, often called catch compare register, and comparing the catch compare register with the incrementing counter value. When the two values matches it will trigger an event such as taking a sample and this would also normally reset the counter value.

Summary of the invention

To achieve at least one of the above objects and also other objects that will be evident from the following description, a method having the features defined in claim 1, and a system having the features defined in claim 11 are provided according to the present invention. Preferred embodiments will be evident from the dependent claims.

More specifically, there is provided according to a first aspect of the present invention a method is provided for performing a wireless synchronization between a master node and at least one group of a vital sign sensors in a sampling measurement arrangement. Each group of vital sign sensors comprising at least one sensor unit, the method comprising: sending out, by said master node, a beacon message for each sensor

    receiving, by a receiver, said beacon message,
    controlling, based on said beacon message, by a control unit, a sampling event in said sensor.

In the essence of the invention, the features provide a synergistic effect that achieves an accurate synchronization for a wireless system comprising a vital sign sensor.

Thereby a method for synchronization of sampling in a measurement is achieved. The beacon message is sent such that each sensor in the group of sensor receives the message at the same time. Thereby, each control unit can simultaneously act on the message to control the sampling event in the sensor. Advantageously an accurate synchronization is achieved wirelessly. Sampling events in the sensors happening at the same time is sought after for any sampling measurement arrangement since this is advantageous in monitoring patients in a hospital, or any other place where vital signs may need to be monitored. The synchronization of the sensor may thus be performed directly when receiving the beacon message. The sampling may occur or be triggered directly upon the sensor receiving the beacon message. Thereby the synchronization of the sensor may be triggered directly by the beacon message.

In some examples, the method further comprises for each sensor:

    retrieving, by the control unit, a sensor unit counter value from a sensor unit counter,
    determining, by the control unit, a difference between the beacon message and said sensor unit counter value,
    upon determining that the difference exceeds a predetermined threshold value, adjusting the sensor unit counter, thereby controlling the sampling event in said sensor.

By having, and adjusting the sensor unit counter, the sampling event can be controlled such that the sampling event occurs at the wanted moment. Some errors in the sampling time may occur naturally, and therefore the synchronization is needed. This example provides a synchronization method that allows for minor error but will synchronize upon the detected error being too large.

In some examples, the beacon message transmitted from said master node comprises a master node counter value, or a known time interval. Advantageously, the sampling synchronization can be controlled in a facilitated manner.

Synchronization of the sampling of a sensor can basically be performed in at least the three ways listed below.

- By sending out beacon messages with a known intermediate time interval, the sensors can synchronize their internal timer to match the known time interval. The sensors can thereafter sample synchronized together or to predetermined time by the master unit.
- By the sensors sampling directly after receiving a beacon message.
- The master node counter value, can be compared to the sensor unit counter value in order to synchronize the sampling event. The comparison can be made either at the sensor or at the master node.

In some examples, the sampling measurement arrangement is an ECG measurement arrangement. The sampling measurement arrangement may be any sampling measurement arrangement. For example the sampling measurement arrangement may comprise blood pressure sensor, respiration sensor or any other sensor. For an ECG measurement arrangement the wireless synchronization enables for measuring and monitoring without the hassle of cables being stuck to the sensors on the patient body. The lack of cables can prevent accidental removal of the sensors if the cable get stuck.

In some examples, the beacon message comprises a reset value, and wherein the method further comprises for each sensor: identifying, by the control unit the reset value, and resetting the sensor unit counter. Advantageously a synchronous reset can be done for all sensors in a group. The restart of the sensors can be done at any time during sampling, and can provide a synchronization of all sensors. The reset command can be used at the start of a measurement in order to ensure accurate measurement. The main function with the reset is that all sensors may start sampling at the same time so that a e.g., a packet counter pointing at the sample values would have the same value for all sensors providing synchronized data pointer.

In some examples, the method further comprises

transmitting, by a transmitter in said sensor, a recorded data to a receiving unit. The recorded data may be a value sensed by the sensor. The recorded data may be a time value for the time of the sampling.

In some examples the receiving unit is said master node. Advantageously, a compact system can be provided. Any suitable device may act as a receiving unit. The data sent to the receiving unit may in some examples be information regarding the controlled sampling event, such as an error detected, or which adjustment was made.

In some examples the method further comprises storing, by a storing unit in said sensor unit, said beacon message, and/or said difference. By storing the beacon message or the difference, the stored values may be used to identify irregularities in the sampling. Thus providing a more accurate synchronization method. It should be noted that any data can be stored in the storing unit. For example, the storing unit may have a value stored in it that can be compared with the beacon message in order to control the sampling event.

In some examples the method further comprises

comparing the determined difference with a stored difference.

In some examples, the group of sensors comprises more than one sensor, and wherein one of said sensors is the master node. Meaning, that one of the sensors may act and have the abilities of the master node. Advantageously a compact system may be provided. If the sensor can act as a master node, a group of sensors may be adapted to perform synchronization amongst themselves.

According to a second aspect, there is provided a system for performing wireless synchronization of sensor units in a sampling measurement arrangement. The system comprising:

a master node;
at least one group of vital sign sensor units, each group comprising at least one sensor unit,
said master node being configured to send out a beacon message to said at least one group of sensor units;
each sensor unit comprising a receiver, the receiver being configured to receive said beacon message,
each sensor unit further comprising a control unit being configured to, upon receipt of said beacon message, control a sampling event in said sensor.

The system provided may have any same advantages as described in relation to the method above.

In some examples the control unit is configured to, upon receipt of said beacon message, determine a difference between said beacon message and a sensor unit counter value. The control unit being configured to adjust the sensor unit counter in the event the difference is over a threshold value, thereby control said sampling event.

In some examples the master node comprises a master node counter, wherein a master node counter value represents a state of the master node counter, and wherein said beacon message comprises said master node counter value or known time interval.

In some examples the group of sensor units comprises three sensor units. The group of sensors may comprise two sensor unit. In other examples the group of sensors may comprise more than three sensor units.

In some examples the sensor unit comprises a storing unit configured to store the determined difference, and/or the beacon message.

Generally, all terms used in the claims are to be interpreted according to their ordinary meaning in the technical field, unless explicitly defined otherwise herein. All references to "a/an/the [element, device, component, means, step, etc]" are to be interpreted openly as referring to at least one instance of said element, device, component, means, step, etc., unless explicitly stated otherwise. The steps of any method disclosed herein do not have to be performed in the exact order disclosed, unless explicitly stated.

Brief description of the drawings

The above, as well as additional objects, features and advantages of the present invention, will be better understood through the following illustrative and non-limiting detailed description of preferred embodiments of the present invention, with reference to the appended drawings, where the same reference numerals will be used for similar elements, wherein:

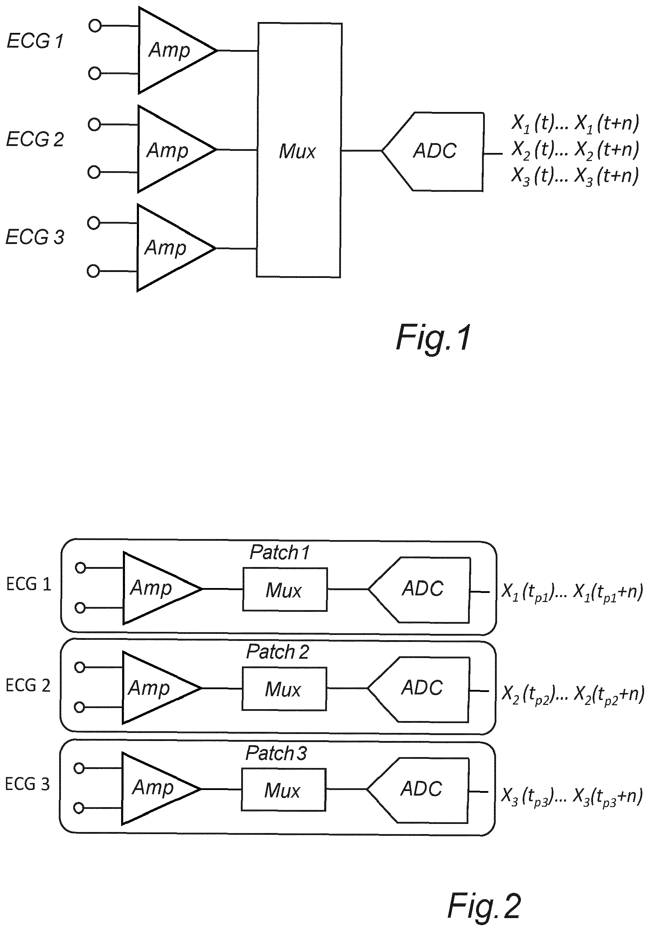

Fig. 1 shows an analog to digital converter having a single MUX.
Fig. 1 shows an analog to digital converter wherein each separate signal is converted by a single MUX.
Fig. 3 shows an example of a diagram of a master node and a group of sensors.
Fig. 4 shows another example of a diagram of a master node and a group of sensors.
Fig. 5 shows a flow diagram of a method according to the disclosure.
Fig. 6 shows a flow diagram of a method according to the disclosure.
Fig. 7 shows a flow diagram of a method according to the disclosure.

Description of embodiments

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and fully convey the scope of the invention to the skilled person.

In an example of performing an ECG, the ECG is measured using a differential amplification of the voltage generated by the heart contracting. These voltages are typically in the mV range while the common mode noise could be in the volt range (50 Hz coupling to mains). As the measurement is performed differentially there may be at least two electrodes that generate one measurement. More than two electrodes may be used, and a common configuration is using five measure electrodes and one ground/right leg drive electrode.

The ECG signal collected by the electrode in the sensor is an analog signal. The analog signal is then converted into a digital signal, called Analog Digital Conversion, ADC.

Fig. 1 discloses an example of system having one sensor comprising one ADC for having three ECG amplifiers resulting in three ECG lead measurements. In Fig. 1 analogue ECG signals 1-3 are converted to X1 to X3 at a defined time t. Thus $X1(t)$ represent the digitalized value of ECG 1 at the given time t. To continuously represent the ECG 1 signal a new value is converted at time t+1. The time difference, between t and t+1, would represent at with period the sampling is taken place and the sampling frequency would then be calculated as 1/period time. Good accuracy of the period can be achieved by using a crystal where it's easy to achieve $10^{-5}$ accuracy (10 ppm).

In the example of ADC in Fig. 1 a MUX is used to switch between signal ECG 1 through ECG 3. In practice, this means that X1 and X2 are not converted at the exact time. Typically, the difference would be the conversion time for one value + the switching time. However, if this time is short enough, compared to the analogue signal spectrum, this would not be noticeable or have any practical disadvantage. Thus, the ADC is designed and selected so that you can assume that $X1(t)$, $X2(t)$ and $X3(t)$ are sampled at the exact same time. A result of this fact makes it possible to digitally combine and process the ECG leads to form e.g. the Wilson central terminal or the augmented limb leads which were originally retrieved by specific analogue amplifier configurations. Another example when this can be used is using the formula for the limb leads.

$$\text{Lead I} + \text{Lead III} = \text{lead II}$$

If the ECG signals $X1(t)$, and $X3(t)$ represent the lead I and III, lead II can be calculated by adding $X1(t)$, and $X3(t)$ together.

Performing the mathematical linear combinations described above requires that the samples of $X1(t)$, and $X3(t)$ are taken at the same time t. If not the combined ECG signal would change in form and the formula (1) would no longer be valid. The synchronization in time is dependent on the frequency content of the signals. The signal ECG 1- ECG 3 contain the ECG component, possible pacemaker pulse and a noise component.

A sought for quality ECG for monitoring is low pass filtered at 150 Hz and sampled at 1000 Hz. The higher sampling rate is for getting the highest frequency components in the QRS complex that otherwise could be missed, causing of a slightly lower QRS complex or variating height due to the missed information.

The QRS complex is the combination of three of the graphical deflections seen on a typical ECG, and represents ventricular depolarization.

Needed synchronization would then be perfectly sufficient with ¼ of the sampling period i.e. 250 uS.

Turning to Fig. 2 which discloses three separate sensor units all including one ADC converting the ECG 1-3 separately which correspond to the present inventive concept.

To achieve this the ADC in the first sensor, the second sensor and the third sensor need to be started at the same time and thereafter periodically controlled to maintain synchronization. Even a very small deviation in sample frequency, between the sensors, may with time grow and eventually cause the data to be unsynchronized.

In the preferred example, the synchronization between tp1, tp2 and tp3 is within ±250uS.

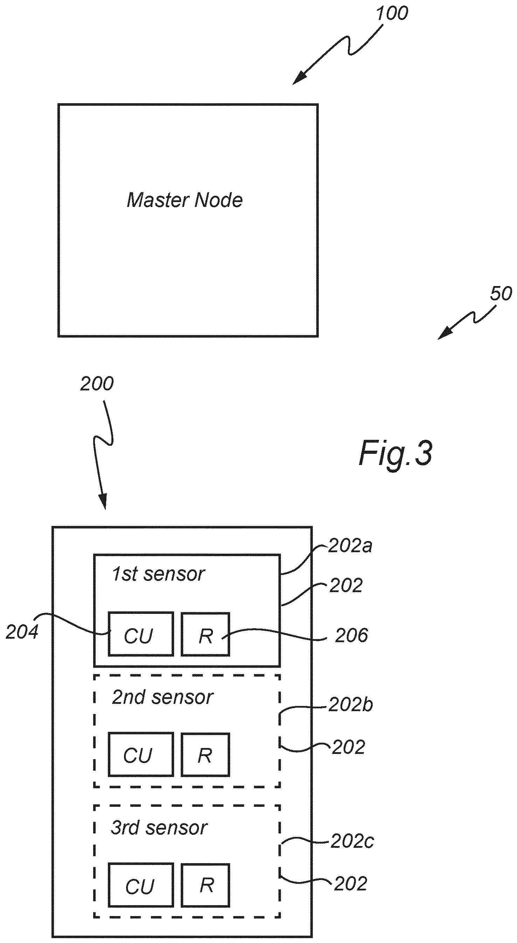

Turning to Fig.3 a wireless system is shown that is configured to perform a synchronize action of a sampling in a vital sign measurement arrangement. It is beneficial to be able to control sampling in terms of the sampling event, and/or sampling time in a vital sign measurement system. The sampling time may be the sampling event. The sampling event may be the time when a sample is taken/sensed by a sensor. Any suitable vital sign sensor that can be used for a sampling measurement arrangement can be used within the inventive concept. For example a vital sign sensor such as an ECG, or a blood pressure sensor or a respiration sensor.

The system is a wireless system, not requiring any cables to perform a synchronization of the sampling in the sampling measurement arrangement. Advantageously the wireless system has less data loss as there are no cables that can get tangled and result in sensors getting pull off from the body. Also, the absence of cable facilitate handling of the patient as the cables are not in the way. Finally, it is may be more convenient for the patient who need long term ECG.

In some instances the sampling measurement arrangement is an electrocardiogram, ECG measurement arrangement. It is important when performing an ECG that the sensors are synchronized in order to provide correct ECG information. Synchronizing the ECG sampling using separate sensors is advantageous to provide accurate ECG that can be used for monitoring. Thereby diseases can be detected in time and thus improved patient safety can be achieved.

In order to detect abnormalities, it is important that all sensors are sampling simultaneously. The provided system and method herein provides synchronization of a measurement system to achieve synchronous sampling

The system comprises a master node 100 and a group of sensors 200. The group of sensors comprises a first sensor 202a, a second sensor 202b and a third sensor 202c. The sensors 202a, 202b, 202c are vital sign sensors such as ECG, blood pressure, or respiration etc. In a preferred example of the disclosed system, the system comprises three sensors 200. The system may comprise only one sensor, preferably more than one sensor.

One sensor may be configured to measure and process more than one vital sign measurement, e.g., measure two ECG leads or pulse oximeter and respiration rate.

The dotted lines displays that the second sensor 202b and third sensors 202c are optional. The system only requires one sensor 202. But the system may comprise more than one sensor, and in some examples, the system may comprise more than three sensors.

In some examples, the system may comprise one master node 100, and more than one group of sensors 200. One master node 100 may be configured to perform the disclosed synchronization method with ten group of sensors. In some examples, the master node may be configured to perform the synchronization method with 10- 50 group of sensors. The master node may be configured to perform the synchronization method with 50-100 group of sensors. Thereby one master node can service synchronization of a plurality of group of sensors, which facilitates synchronization, and measurement of vital signs.

The sensor comprises a control unit 204, and a receiver 206.

The master node 100 is configured to send out a beacon message, that can be received by the receiver 204 in each sensor 202 in a group of sensors 200. After receiving the beacon message, the control unit 202 can control the sampling event in the vital sign sensor.

The beacon message sent out by the master node 100 comprises instructions for how to control and/or adjust the sensor.

The beacon message may be sent to all group of sensors at the same time in examples wherein the system comprises more than one group of sensors.

For example, the control unit 204 in the sensor 202 may control the sampling such that sampling occurs at the time of receiving the beacon message. The control unit 204 may in other examples control the sampling at any other time given by the beacon message. The control unit 204 may perform an adjustment to the sampling event. The adjustment may be adjusting the timing of the sample, or for example the extent of the sample taken.

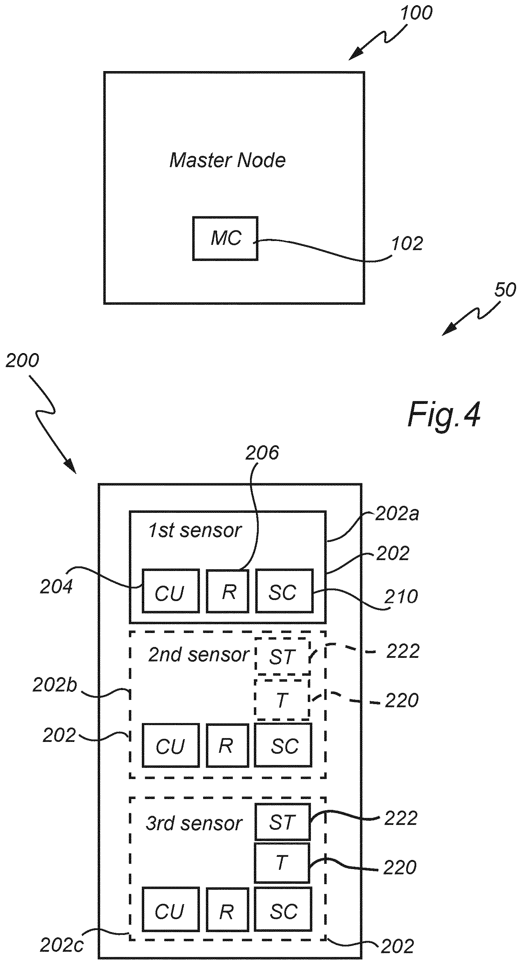

Fig. 4 show an example in which the sensor 200 comprises a sensor counter unit 210. The sensor counter unit 210 has a sensor counter value, which can be used when comparing the beacon message. By adding the sensor counter unit 210, the sensor 200 may have a continuous counter that can improve accuracy of the synchronization.

In some cases, the master node 100 may comprise a master node counter 102, having a master node counter value.

Each sensor 202a, 202b, 202c may have the sensor counter unit 210 connected to a crystal. When the sensor counter unit reaches a threshold overflow value the counter can be restarted. The counter could be reset or started by a radio message with a start command. In the sensor, the local sensor counter value could continuously be compared with the incoming beacon messages. Said incoming beacon messages may have an exact periodic timing. The threshold overflow value can be corrected with a deviation/error in whole number of clock cycles of the sensor counter unit used for counter. This procedure makes it possible to regulate the sensor unit counter continuously. Typically, each received beacon message only affect the mean error partly, i.e. the error is calculated as the mean deviation from several received beacon messages. The beacon message could in some examples include a counter for the handling of missed beacon messages.

In order to achieve a good synchronization, it is advantageous to keep control or keep track of the sampling events to know if two samples were taken at the same time or not. In order to compare sample values it is beneficial to compare the samples takes at the same time, or known time differences.

One or more sensors 202a, 202b, 202c may comprise a sample counter and/or a packet counter. The sample counter may control the sampling so that each sampling is given a sample counter value. The sample counter value may indicate which sample is taken, and may be compared to the sample counter values in the other sensors. The sample counter value may be dependent on time t and t+1 for describing the sampling event.

This is beneficial in order to keep track of the sampling, to know if two samples taken from two different sensors are taken at the same time. The sample counter value may be sent to a receiver together with the sample. The data may be processed in order to control the beacon messages to be sent out in order to improve accuracy of the synchronization.

A packet counter may be used to send a packet counter value for a block of data sent to the receiver.

When the sensor is reset, the sample counter and/or packet counter may be reset. The sample counter and/or packet counter may be a separate unit.

The sensor 200 may comprise a transmitter 220. The transmitter may be configured to transmit a data to a receiving unit. The receiving unit may be comprised in the master node. In some other examples the receiving unit is part of a computer system. The receiving unit, is configured to receive a value or data from the sensor. The receiving unit may be configured to receive the beacon message.

The sensor 222 may comprise a storing unit 222. The storing unit may be configured to store a data or a value. The storing unit may be configured to store the beacon message. The storing unit may be configured to store the data collected in the sampling event. In some examples, the determined difference may be stored in the storing unit.

The control unit 204 may further be configured to retrieve the stored data or value to perform a calculation or comparison between the stored data and a received beacon message.

It should be noted that the transmitter 220 and the storing unit 222 are optional features.

All sensors 202 in a group of sensors may comprise a transmitter 220 and/or a storing unit 222. In some examples one or more of the sensors comprises a transmitter 220 and/or a storing unit 222.

The synchronization method may require high accuracy of sending beacon messages and locking a counter time when the beacon message is received. Otherwise one clock cycle accuracy is achievable. However, depending on hardware platform, e.g. one byte hardware counter, a crude synchronization may be beneficial before entering a clock counter regulation method. This can be achieved by using larger software counters, with less accuracy, but with ability to measure for longer time periods. In such a scenario the hardware counter may be counting to a raw threshold.

Another way of synchronizing the sensors would be that one sensor is used as a reference for the other sensors. The master sensor radio message may then contain information about the clock counter and the corresponding data sample counter.

Another way of synchronizing the sampling in the is to sample directly after receiving a specific beacon message. Thus, this sample would be synchronized to the received beacon message. Typically, such an implementation would fit data with lower sampling rates and lower synchronizations demands.

There exist several techniques for noise reduction where the noise is removed mathematically after the ECG signals have been sampled. The method described herein may include oversampling strategies. For example initially sampling at a much higher sample rate than the properties of the ECG signal with the intention of sampling noise with higher frequency content and then mathematically removing them. If oversampling is used the synchronization need could increase to the 1/(oversampling frequency). However, if the technic is only using the same signal, e.g. oversampling at 10 000Hz directly follow by the mean average of 10 and down sampling, the requirement of synchronization may be reduced.

The ECG can be used to measure the rate and rhythm of heartbeats, the size and position of the heart chambers, the presence of any damage to the heart's muscle cells or conduction system, the effects of heart drugs.

In some examples, the wireless sensors 202 may perform a synchronization between themselves. In such cases one of the sensors sends a signal to the other sensors and synchronization can be achieved in the same manner that the methos and system functions with the master node. Hence, the sensor may act as the master node.

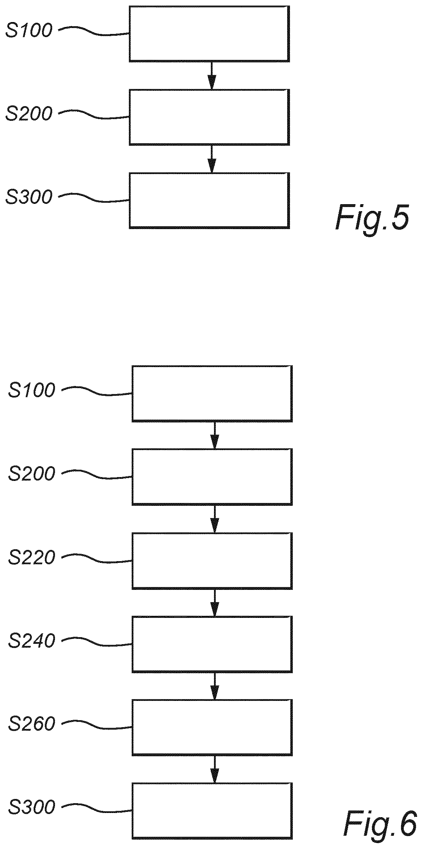

Fig. 5-7 shows flow diagrams of methods according to the disclosure.

In Fig. 5, the steps of sending S100 a beacon message, which is performed by the master node is followed by receiving S200 the beacon message at the receiver in the sensor 200, whereafter the control unit 204 may control S300 the sampling event.

The flow diagram of Fig. 6 further shows the optional step of retrieving S220 which is also performed by the control unit 204 a sensor unit counter value from a sensor unit. The control unit 204 may in some examples retrieve a sample counter value and thereafter control S300 said sampling event based on the sample counter value. The method may optionally comprise a step of determining S240 a difference between the beacon message and the retrieved value form the sensor 200. In some examples the difference is determined based on the beacon message itself, in some examples the beacon message comprises a beacon message value that is used in determining S240 the difference. The method may also comprise the step of adjusting S260 the sensor unit counter if the determined difference is above a predetermined threshold value. The predetermined threshold value is preferably set to increase accuracy of the synchronization.

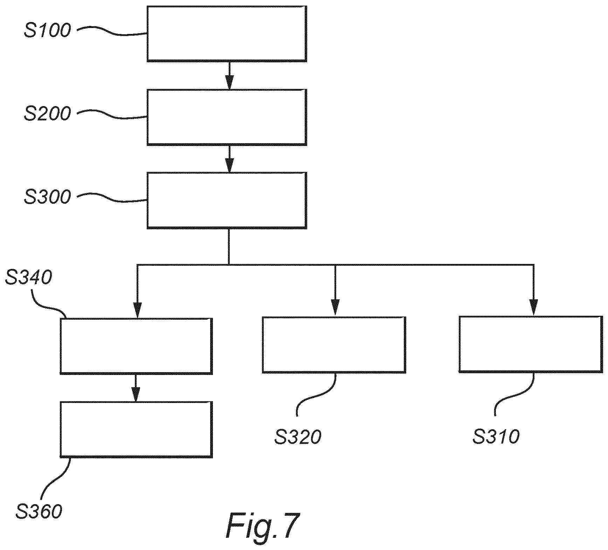

Turning to Fig. 7, a further method is disclosed. The flow diagram shows the same steps as Fig 5, and in addition, some optional steps. The method may comprise identifying S310 a reset value that is comprised in the beacon message. The control unit 204 identifies if the beacon message comprises the reset value, and thereafter the control unit 204 may reset the sensor unit counter 210.

The sensor is preferably arranged to record a data from the sampling event. In some examples, a recorded data may be transmitted (S320) by a transmitter in said sensor 200 to the receiving unit. In some examples, the master node comprises the receiving unit. In other examples, the master node may be the receiver. In some examples, the receiver is a separate unit, which may be part of a computer system.

The method may comprise the step of storing S340 data or a value. A Storing unit which may be located in the sensor 200 may store a recorded data, and/or the beacon message, and/or the determined difference or any other value that may be used in controlling the sampling event.

When the differences is stored, the method may comprise the step of comparing S360 the determined difference with the stored difference in order to determine if the difference exceeds the difference in the stored difference.

It should be noted that the method does not need to comprise all steps and that some steps are optional. The optional steps shown in Fig. 7, may be applied, each on their own, to either the method shown in Fig. 5, or Fig. 6.

The same logic applies to the optional steps disclose din Fig. 6, that some optional step may be applied in addition to the steps shown in Fig. 5.

The master node may be connected to a computer comprising a processor. The processor may be configured to separate out the received data and sorting it to each respective group of sensors.

It will be appreciated that the present invention is not limited to the embodiments shown. Several modifications and variations are thus conceivable within the scope of the invention which thus is exclusively defined by the appended claims.

The methods described herein of performing a wireless synchronization between a master node and at least one group of a vital sign sensors can be automatically be performed by a computer. Hence the method can be a computer implemented program comprising the instructions to perform any mentioned step herein.

**Claims**

1. Method of performing a wireless synchronization between a master node and at least one group of a vital sign sensors in a sampling measurement arrangement, each group of vital sign sensors (200) comprising at least one sensor unit, the method comprising:
   sending out (S100), by said master node (100), a beacon message for each sensor

   receiving (S200), by a receiver (206), said beacon message,
   controlling (S300), based on said beacon message, by a control unit (204), a sampling event in said sensor (200).

2. The method of claim 1, further comprising

   for each sensor:
   retrieving (S220), by the control unit (204), a sensor unit counter value from a sensor unit counter (210),
   determining (S240), by the control unit, a difference between the beacon message and said sensor unit counter value,
   upon determining (S240) that the difference exceeds a predetermined threshold value, adjusting (S260) the sensor unit counter (210), thereby controlling (S300) the sampling event in said sensor.

3. The method of claim 1 or 2, wherein said beacon message transmitted from said master node comprises a master node counter value, or a known time interval.

4. The method of claim 1 wherein sampling event in said sensor is directly triggered by the beacon message.

5. The method of any one of the preceding claims wherein the sampling measurement arrangement is an ECG measurement arrangement.

6. The method of any one of the preceding claims wherein said beacon message comprises a reset value, and wherein the method further comprises for each sensor: identifying (S310), by the control unit the reset value, and resetting the sensor unit counter and /or a sample counter.

7. The method of any one of the preceding claims further comprising Transmitting (S320), by a transmitter in said sensor, a recorded data to said a receiving unit.

8. The method of claim 6, wherein said receiving unit is said master node.

9. The method of any one of the preceding claims, wherein the group of vital sign sensors comprises more than 1 sensor, and one of said sensors is said master node.

10. The method of any one of the preceding claims further comprising, storing (S340), by a storing unit in said sensor unit, said beacon message, and/or said difference.

11. The method of claim 10, wherein the method further comprises Comparing (S360) the determined difference with a stored difference.

12. A system (50) for performing wireless synchronization of sensor units in a sampling measurement arrangement, said system comprising:

a master node (100);
at least one group of vital sign sensor units (200), each group comprising at least one sensor unit (200a, 202b, 202c),
said master node (100) being configured to send out a beacon message to said at least one group of sensor units (200);
each sensor unit (200a, 202b, 202c) comprising a receiver (206), the receiver being configured to receive said beacon message,
each sensor unit (200a, 202b, 202c) further comprising a control unit (206) being configured to, upon receipt of said beacon message, control a sampling event in said sensor (200a, 202b, 202c).

13. The system (50) of claim 12, wherein said control unit (206) is configured to, upon receipt of said beacon message, determine a difference between said beacon message and a sensor unit counter value, said control unit (206) being configured to adjust the sensor unit counter in the event the difference is over a threshold value, thereby control said sampling event.

14. The system (50) of claim 12 or 13, wherein said master node comprises a master node counter, wherein a master node counter value represents a state of the master node counter, and wherein said beacon message comprises said master node counter value or known time interval.

15. The system (50) of claim 12-14 wherein said group of sensor units comprises three sensor units.

16. The system (50 of any one of claims 12-15, wherein said sensor unit comprises a storing unit (222) configured to store the determined difference, and/or the beacon message.

*Fig.1*

*Fig.2*

Fig.3

*Fig.4*

Fig.5

Fig.6

S100

S200

S300

S340

S360

S320

S310

Fig.7

Europäisches
Patentamt

European
Patent Office

Office européen
des brevets

# EUROPEAN SEARCH REPORT

**Application Number**

EP 23 21 8952

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/240614 A1 (ACQUISTA ANGELO JOSEPH [US] ET AL) 3 August 2023 (2023-08-03) * abstract; figures 1,16,17 * * paragraph [0003] – paragraph [0004] * * paragraph [0018] * * paragraph [0037] – paragraph [0044] * * paragraph [0099] – paragraph [0108] * ----- | 1-16 | INV. G16H40/60 A61B5/00 H04W56/00 |

TECHNICAL FIELDS
SEARCHED (IPC)

G16H
A61B
H04W

The present search report has been drawn up for all claims

| Place of search | Date of completion of the search | Examiner |
|---|---|---|
| Munich | 17 April 2024 | Gardiner, Alexander |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 21 8952

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2024

| Patent document cited in search report | Publication date | Patent family member(s) | Publication date |
|---|---|---|---|
| US 2023240614 A1 | 03-08-2023 | AU 2017231808 A1 | 04-10-2018 |
| | | CA 3017199 A1 | 14-09-2017 |
| | | CN 109069005 A | 21-12-2018 |
| | | CN 114190896 A | 18-03-2022 |
| | | EP 3426139 A1 | 16-01-2019 |
| | | EP 4245359 A2 | 20-09-2023 |
| | | MY 191412 A | 27-06-2022 |
| | | SG 11201807715U A | 30-10-2018 |
| | | US 2017258402 A1 | 14-09-2017 |
| | | US 2023240614 A1 | 03-08-2023 |
| | | WO 2017156246 A1 | 14-09-2017 |

EPO FORM P0459

For more details about this annex : see Official Journal of the European Patent Office, No. 12/82